# EUROPEAN PATENT SPECIFICATION

(11) **EP 1 471 962 B1**
(45) Date of publication and mention of the grant of the patent: **12.03.2014**
(21) Application number: 03715867.2
(22) Date of filing: 03.02.2003
(51) Int. Cl.: A61M 16/06, A61M 16/10, A61M 16/16

(54) **BREATHING ASSISTANCE APPARATUS**
GERÄT ZUR UNTERSTÜTZUNG DER ATMUNG
DISPOSITIF D'ASSISTANCE RESPIRATOIRE

(30) Priority: 04.02.2002 NZ 51703102
(43) Date of publication of application: 03.11.2004
(60) Divisional application: 14150364.9
(73) Proprietor: Fisher & Paykel Healthcare Limited, East Tamaki, Auckland 1006 (NZ)
(72) Inventor: MACKIE, Scott Robert, Ponsonby, 1002 Auckland (NZ); WHITE, Craig Karl, Remuera, 1005 Auckland (NZ); FOREMAN, Mark, One Tree Hill, 1003 Auckland (NZ)
(74) Representative: Hoarton, Lloyd Douglas Charles
(86) International application number: PCT/NZ2003/000012
(87) International publication number: WO 2003/066145

(56) References cited:
- WO-A1-00/72905
- WO-A1-86/02276
- WO-A1-92/20392
- WO-A1-98/57691
- FR-A1- 2 638 361
- US-A- 3 807 445
- US-A- 4 686 354
- US-A- 5 558 084
- US-A- 6 050 260
- US-B1- 6 332 462

## Description

### FIELD OF INVENTION

The present invention relates particularly, though not solely, to the delivery of high flow humidified pressurised oxygen and/or air by way of a wide bore nasal cannula to a patient in need of respiratory support.

### SUMMARY OF THE PRIOR ART

There are two common methods for delivering oxygen to a patient. The simplest method is via a Venturi mask. This method has the advantage of delivering, precisely and constantly, the desired level of oxygen, or fraction of inspired oxygen (FiO2), provided that the FiO₂ is less than 50%. The operation of a Venturi mask is based on the Bernoulli principle. One hundred per cent oxygen flowing through the narrow orifice results in a high-velocity stream that entrains room air through multiple open side ports at the base of the mask or on top a humidifier. The amount of room air entrained to dilute the oxygen depends on the orifice size. Venturi masks can provide FiO₂ levels from 24-50% with great accuracy.

The other method of oxygen delivery is dual prong nasal cannula e.g. see WO 86/02276. Nasal cannula generally consist of an entry tubing, either symmetric or single sided that lies across the upper lip. Protruding from this tubing are open ended prongs which extend into the nares of the patient to deliver oxygen. Nasal cannula have the advantage of being more comfortable and acceptable than a face mask to most patients. When using nasal cannula, however, FiO₂ cannot be precisely controlled because it is affected by the route of inhalation (nose or mouth), upper airway geometry and breathing pattern.

Other, less popular, methods for oxygen delivery are used when a FiO₂ higher than 50% is required. A non-rebreathing mask with reservoir and one-way valve may deliver a FiO₂ of up to 90%, provided that leaks around the mask have been eliminated by tight seals. These masks are rarely used because they are not easily accepted by patients and carry significant risk of the patient rebreathing expired air. The rebreathing of expired air is undesirable as this air contains high levels of carbon dioxide.

As oxygen is supplied as a dry gas it is well known in the art to either heat and/or humidify gases before delivering them for breathing by a patient. In particular when delivering oxygen, or oxygen / air mixture, it has proven beneficial to humidify the gases first. In WO 01/41854 of Vapotherm, Inc. a system is disclosed that allows the delivery of humidified oxygen through a nasal cannula. This system uses a narrow bore conduit and nasal cannula with a high resistance to gas flows, thereby requiring the oxygen be of a high pressure. Air, as well as oxygen, can also be passed down the conduit and nasal cannula and it too must be of a high pressure. This system allows the delivery of high flows of oxygen enriched air to the patient, but is limited in the flows achievable due to the narrow bore of the cannula resulting in high resistance gas flow and excessive velocity and noise upon exiting the cannula. Furthermore, the narrowness of the nasal cannula in this system allows easy expiration of gases between the prongs and nares and therefore does not create any positive airway pressure.

Innomed Technologies, Inc. manufactures a nasal cannula device called the NASALAIRE™. In this device air or oxygen travels down a wide bore conduit to nasal cannula. The NASALAIRE™ creates a physical seal between the nares and itself, and relies on the absence of leaks around itself and the nares to deliver pressure supplied by a continuous positive airway pressure (CPAP) blower to the airway of the wearer. The wearer is required to breathe out and in of the NASALAIRE™, thereby rebreathing some of the exhaled air from the lungs. The NASALAIRE™ is not designed to deliver humidified gases to the patient, being unheated and without insulation of any type.

WO00/72905 discloses a nasal cannula comprising nasal prongs which are configured to have a snug fit with the nares of a patient.

### SUMMARY OF THE INVENTION

It is an object of the present invention to provide a breathing assistance apparatus which goes someway to overcoming the above mentioned disadvantages or which will at least provide the public a useful choice.

Accordingly the present invention consists in a breathing assistance apparatus comprising:
a pressurised source of gases,
humidification means (1) adapted to, in use, be in fluid communication with said source of gases and adapted to in use humidify said gases,
humidified gases transport means (6) adapted to, in use, be in fluid communication with said humidification means (1) and adapted to in use convey said humidified gases,
heating means (10) disposed within said transport means (6) and adapted to in use heat said humidified gases as they pass through said transport means (6), and
nasal cannula (30), adapted to, in use, be in fluid communication with said transport means (6) and to deliver said humidified gases to a patient, said nasal cannula (30) including at least one wide bore nasal prong (40, 41) that does not seal the nares and that allows high flow delivery of said humidified gases and creates positive airway pressure in said patient's airway during inspiration and positive end expiratory pressure during expiration.

### BRIEF DESCRIPTION OF THE DRAWINGS

One preferred form of the present invention will now be described with reference to the accompanying drawings in which;
**Figure 1** is an illustration of a respiratory humidifier system that may be used with the nasal cannula of the present invention,
**Figure 2** is an illustration of the humidifier base of the respiratory humidifier system of Figure 1,
**Figure 3** is a side view of the nasal cannula of the present invention in use by a patient,
**Figure 4** is a front perspective view of the nasal cannula of the present invention,
**Figure 5** is a back perspective view of the nasal cannula of the present invention,
**Figure 6** is a further back perspective view of the nasal cannula of the present invention, and
**Figure 7** is a side perspective view of a further embodiment of the nasal cannula of the present invention.
**Figure 8** is a side view of nasal prongs showing hidden details and one embodiment of a cannula with pressure relief valve.
**Figure 9** is a cross-sectional side view of the pressure relief valve as shown in Figure 8 when in operation.

### DETAILED DESCRIPTION OF THE PREFERRED EMBODIMENTS

Whether used in a hospital environment or in a home environment, the nasal cannula of the present invention will generally have associated three main pieces of apparatus. Firstly an active humidifier that controls the temperature of a heater plate heating a body of water to achieve a desired temperature and humidity of the gases being humidified. Secondly a transport conduit from the humidifier to the patient is also required, which is preferably heated to reduce condensation, or "rain out". Thirdly a cannula designed to fit up into the nasal cavity and deliver humidified, pressurized gases.

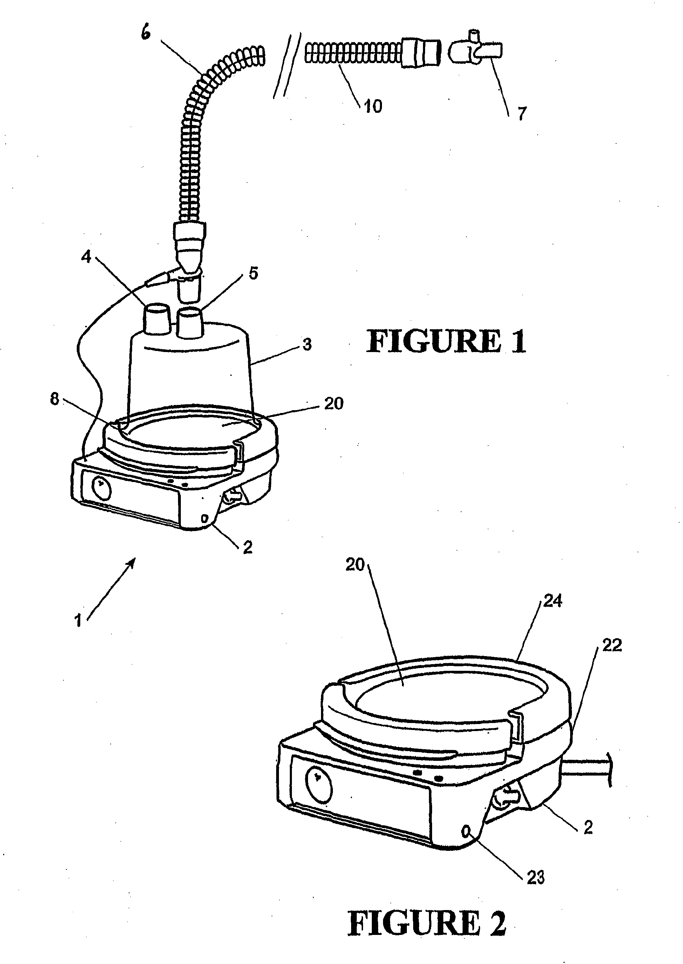

Referring to Figure 1 a humidifying apparatus as might be used in a hospital generally referenced 1 is shown. The apparatus comprises a body 2 containing heating means comprising a heating plate 20 having an electric heating element therein or in thermal contact therewith and control means for example electronic circuitry which may include a microprocessor for controlling the supply of energy to the heating element. The body 2 is removably engageable with a humidifying chamber 3 that contains water for humidifying gases.

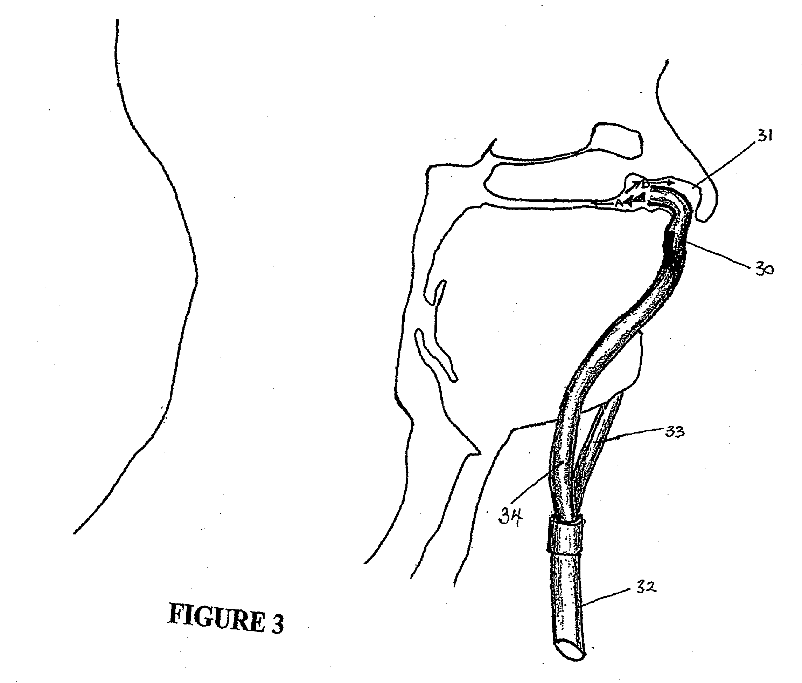

Referring to Figure 2, which shows the humidifier apparatus in more detail, the humidifying chamber 3 has edges, which engage with collar 24 on the humidifier apparatus. The gases to be humidified may be a mixture of air, oxygen and anaesthetic for example, which are supplied to the chamber through gas inlet 4. This might be connected to a ventilator, source of pressurised oxygen, flow generator, or air compressor. A gases outlet 5 is also provided and the gases outlet 5 is connected to the conduit 6 (Figure 1), which conveys humidified gases to the patient at the end 7 of the conduit. The end 7 of the conduit has a cannula (30, in Figure 3) connected to the patients nose so as to supply humidified gases to the user. The humidifier heater plate 20 has a temperature transducer 8 that is in electrical connection with the electronic control circuitry in body 2 of the apparatus so that the control means monitors the temperature of the heating plate.

A heating element 10 is provided within the conduit 6 to help prevent condensation of the humidified gases within the conduit. Such condensation is due to the temperature of the walls of the conduit being close to the ambient temperature, (being the temperature of the surrounding atmosphere) which is usually lower than the temperature of the humidified gases within the conduit. The heater element is effectively replaces the energy lost from the gases through conduction and convection during transit through the conduit. Thus the conduit heater element ensures the gases delivered are at an optimal temperature and humidity.

### Nasal Cannula

The nasal cannula 30 of the present invention is shown in side view in Figure 3 when located within a patient's nares 31. One end of the inlet tube 32 is attached to end 7 of the conduit 6 (see Figure 1) and accepts the gases flow from the pressurised source supplying gases and the humidifier. The other end of the tube 32 terminates at a y-connection where each of the arms of the Y are tubes 32, 33. These tubes connect to the two nasal prongs of the cannula. The diameter of the inlet tube 32 and tubes 33, 34 are as large as possible to ensure minimal pressure drop in the gases before delivery to the patient.

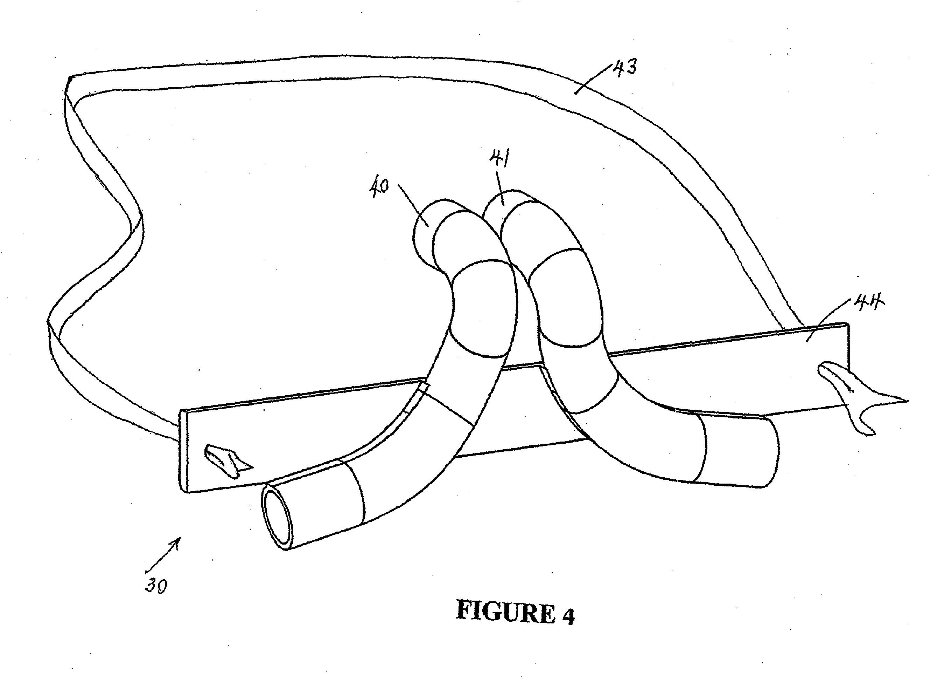

Referring to Figures 3 to 6, operatively attached to a body 44 are two nasal prongs 40, 41. Gases flow from the inlet tube 32 that branches, at a y-connection, into two tubes 32, 33 and these tubes are operatively attached to the nasal prongs 40, 41. Each of the prongs 40, 41 extend into a corresponding nare of the patient. The prongs 40, 41 and body 44 are attached to the patient's face via a strap 43. The strap 43 is attached to the body by appropriate fastening means, for example, VELCRO^{™}, which may be adjusted to suit the size of the patients head and ensures the prongs 40, 41 remain within the patient's nares and body 44 fits comfortably against the patient's face.

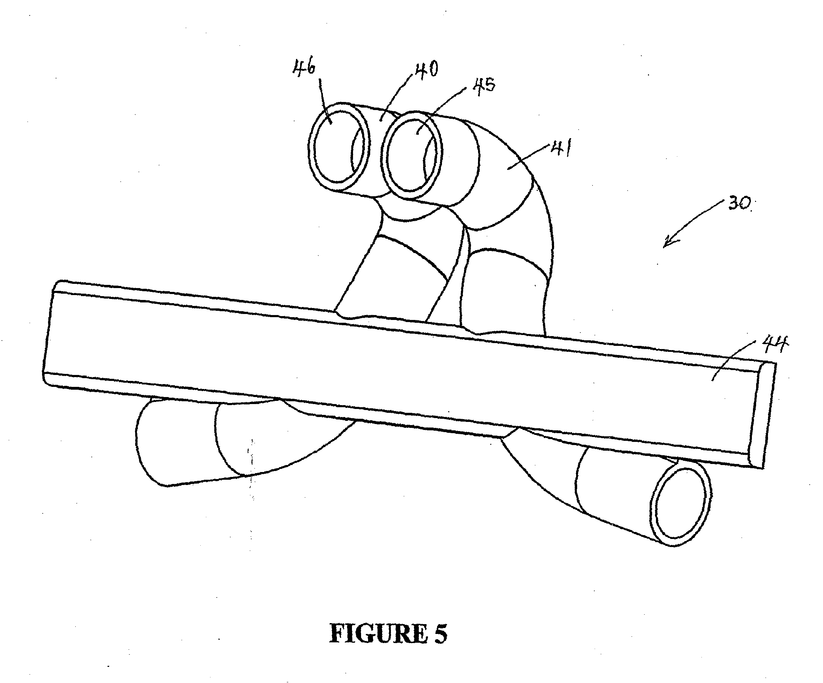

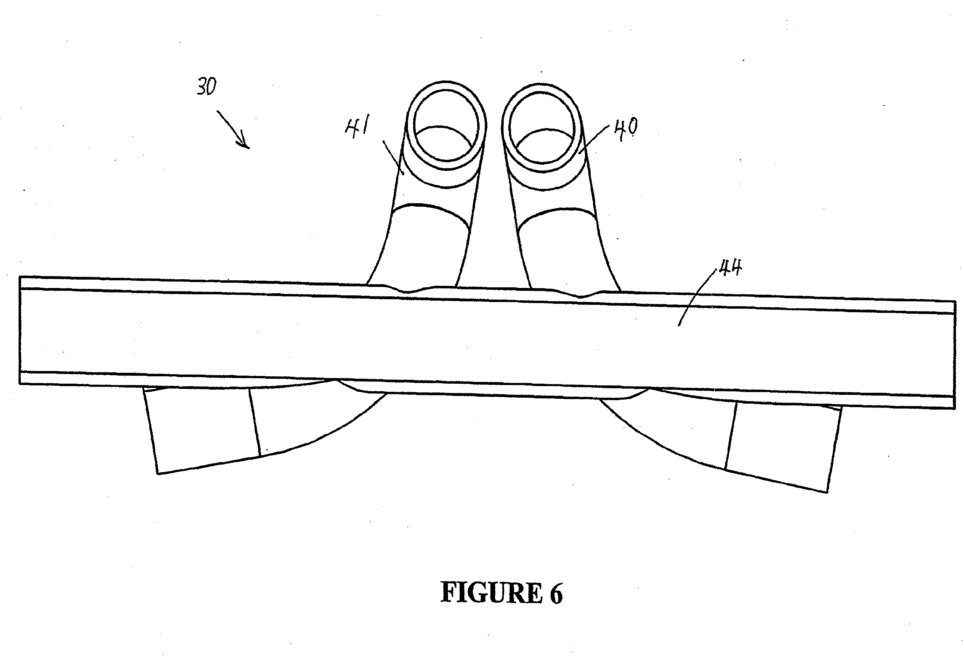

Referring to Figures 5 and 6, the prongs 40, 41 are angled inwards toward the septum of the nose meaning the prongs are anatomically correct and follow the nasal cavity towards the posterior (as can also be seen in Figure 3).

The prongs 40, 41 themselves are round or elliptical in shape or may be shaped to correspond to the inner dimensions of the nasal cavity. The diameter of each of the cannula is chosen such that exhaled gases can be breathed out by the patient past the each of the prongs, as indicated by arrows A and B in Figure 3, where arrow A shows the direction of inhaled gases and arrow B shows the direction of exhaled gases.

In the embodiment where the prongs 40, 41 follow the inner shape of the patient's nostrils, a more efficient flow of gases into the patient's lungs is achieved. With the cannula shaped in this manner, gases flows are directed down the main nasal passage more accurately. Particularly, each of the prongs 40, 41 are curved towards the sagittal (midline) plane, that is, towards the septum of the nose. Furthermore, the top 45, 46 of each cannula points, in use, toward the back of the patient's head.

The entire assembly of the nasal cannula, including the cannula, body and prongs attaching the cannula to the patient's face and nares, are moulded from silicon or other flexible material as are known is the art for cannula construction.

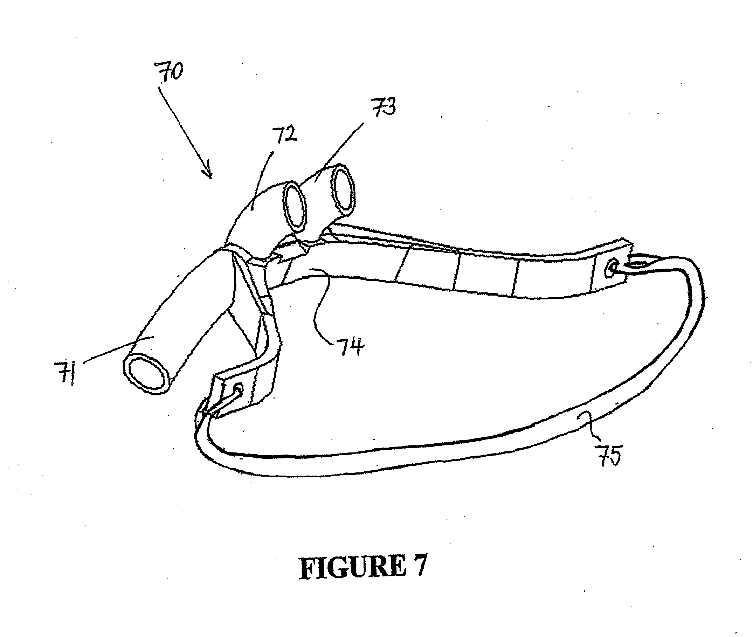

In a further embodiment of the present invention, as shown in Figure 7, the inlet to the cannula 70 is provided through one tube. Here, gases flow from the inlet tube 71 through each of the prongs 72, 73 and into the corresponding nares of the patient. The cannula of this embodiment is also attached to a body section 74 that is shaped so it fits the contours of the patient's face. The cannula and body is maintained against the patients face again by way of a head strap 75.

The cannula of the present invention according to one embodiment comprises wide bore cannula to minimise the flow resistance and the entry velocity. For adults an approximate nare diameter of 6mm has been found to be suitable (actual diameter depends on cannula shape), this compares with 2-3mm in the prior art. This allows the present invention to deliver higher than 30 L/min of oxygen enriched gases, whereas prior art systems with small bore cannula can only deliver an absolute maximum of 6 L/min of dry gas or 20 L/min humidified gas, for the following reasons:
a) Flows higher than 20L/min through existing cannula (i.e. VAPOTHERM™ system) are noisy due to the creation of turbulent gas flow.
b) Existing cannula (i.e. VAPOTHERM™ system) have a high resistance to gas flow, requiring the use of a supply of gas exceeding 50cmH₂O pressure.
c) Flows higher than 20L/min through existing narrow bore cannula (i.e. VAPOTHERM™ system) create a jet of gas upon exiting the cannula that becomes irritating to the airway over short periods of time.

The expiration of gases by the patient against the high incoming flow provides positive end expiratory pressure (PEEP). PEEP keeps the airways and alveoli from collapsing at end-expiration and can reopen airways and alveoli that have already collapsed. This improves gas exchange (decreased intra pulmonary shunt), reduces the resistance to airflow (lung resistance), and makes the lungs less stiff (increased lung compliance). Levels of oxygen and carbon dioxide may improve, reducing the need for supplemental oxygen and the sensation of breathlessness. PEEP may also improve cardiac performance by increasing mean intra thoracic pressure. PEEP is of special advantage to assisting in the treatment of obstructive lung diseases and heart failure, including emphysema, bronchiectasis, chronic bronchitis, cystic fibrosis and pulmonary edema.

The wide bore cannula of the present invention also allows for the provision of gases to the patient that exceeds the patient's peak inspiratory flow. Consequently, a small amount of positive pressure is also generated during the inspiratory phase. This will create inspiratory positive airway pressure (IPAP) that, like PEEP, keeps airways and alveoli from collapsing and reduces the effort to inhale. IPAP is of special advantage to patients who experience breathlessness during respiratory failure.

The ability of the cannula of the present invention to provide these forms of pressure support also allows the cannula to deliver pressure oscillations to the patient. Pressure oscillations are known to improve the clearance of sputum from the lungs and the exchange of respiratory gases between the blood and alveolar air.

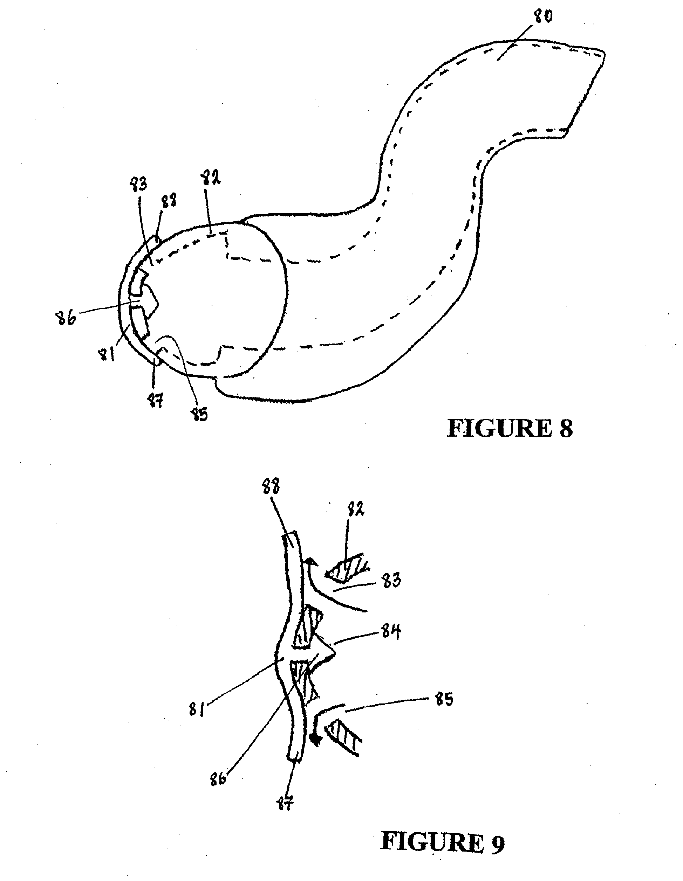

The cannula of the present invention does not create a seal against the nares, and so allow a continuous leakage of gas out of the nose between the cannula and nares. In the event of improper fitting causing the prongs to seal against the nares, one embodiment of the nasal cannula of the present invention is provided with a pressure relief valve. This valve will ensure barotrauma is not inflicted upon the patient. This pressure relief could be similar in form to a CPAP valve as is known in the art or could be constructed integrally within the moulding of the nasal cannula as shown in Figure 8. The continuous flow around the nares allowed by the device not sealing also eliminates the need for a bias flow outlet such as that incorporated in the NASAL-AIRE^{™} manufactured by Innomed Inc.

Referring now to Figures 8 and 9, one embodiment of nasal cannula with prongs 80 is shown (without additional head straps and the like) that has a pressure relief valve 81. The pressure relief valve 81 is a removable flap that is located against the manifold 82 of the cannula. The relief valve 81 is preferably made from a soft flexible material, such as silicone. The manifold 82 has three recesses 83, 84, 85 located in it. In use, the middle 84 of these recesses has located within it an arrow shaped protrusion 86 that forms part of the relief valve 81. The protrusion 86, being made of a soft flexible material can be pushed through recess 84, in order to locate the valve 81 in the manifold. The shape of the protrusion is such that a large pulling force on the valve 81 is required before the protrusion 84 can be removed from the manifold 82. As the ends 87, 88 of the flap 81 are made from a supple material they are capable of being pushed outwards (as shown in Figure 9), when the cannula is in use and when excessive pressure exists in the manifold or prongs, to allow gases to be released through the side recesses 83, 85 on the manifold.

It must be appreciated that Figures 8 and 9 merely show one embodiment of nasal cannula with a pressure relief valve. Other embodiments with pressure relief valves, for example, embodiments including pressure relief valves at the humidification means, humidified gases transport means or elsewhere on the nasal cannula are envisaged.

The cannula of the present invention does not require rebreathing of expired gases; rather the cannula reduces anatomical dead space by flushing the pharynx with fresh respiratory gases. Further, the nasal cannula of the present invention does not deliver continuous positive airway pressure, but instead delivers a form of bi-level positive airway pressure in which PEEP is greater than IPAP.

A further consequence that is provided by the nasal cannula in allowing for the exceeding of peak inspiratory flow is that all gases the patient is breathing are being delivered from the cannula and do not contain any portion of room air as in the prior art. This allows the oxygen percentage in the patient's breath to be controlled over the full range (up to 100%) to be known. This has previously only been possible with a mask, which is much more claustrophobic and restrictive to the patient and severely hinders the patients' ability to talk or eat. The cannula, therefore, incorporates the advantages of Venturi mask, nasal cannula, and non-rebreathing mask as stated earlier without the disadvantages of discomfort (Venturi mask), inconsistent FiO2 (prior art nasal cannula), need for a tight seal between mask and patient (non-rebreathing mask), and possibility of rebreathing expired gases (non-rebreathing mask).

Finally, in summary, the nasal cannula of the present invention allows for the delivery of humidified air (whether the air is blended with or without oxygen) to a patient at flows greater than 30 L/min that offers the following benefits over standard cannula.
1) High FiO₂ (fraction of inspired oxygen), where normal cannula provide less than 32%.
2) Known FiO₂ that is equivalent to a facemask.
3) PEEP (positive end expiratory pressure) caused by breathing out against the inward flow of gases.
4) IPAP (inspiratory positive airway pressure) where delivered flow is greater than the peak inspiratory flow.
5) Delivers pressure oscillations.
6) Reduces breathlessness.

## Claims

1. A breathing assistance apparatus comprising:
a pressurised source of gases,
humidification means (1) adapted to, in use, be in fluid communication with said source of gases and adapted to in use humidify said gases,
humidified gases transport means (6) adapted to, in use, be in fluid communication with said humidification means (1) and adapted to in use convey said humidified gases,
heating means (10) disposed within said transport means (6) and adapted to in use heat said humidified gases as they pass through said transport means (6), and
nasal cannula (30), adapted to, in use, be in fluid communication with said transport means (6) and to deliver said humidified gases to a patient, said nasal cannula (30) including at least one wide bore nasal prong (40, 41) that does not seal the nares and that allows high flow delivery of said humidified gases and creates positive airway pressure in said patient's airway during inspiration and positive end expiratory pressure during expiration.

2. A breathing assistance apparatus according to claim 1 wherein said cannula (30) is provided with two nasal prongs (40, 41) that do not seal within the nares of said patient.

3. A breathing assistance apparatus according to claim 2 wherein said nasal prongs (40, 41) are shaped so they are anatomically correct to said patient's nasal passage.

4. A breathing assistance apparatus according to any one of claims 2 or 3 wherein said nasal prongs (40, 41) are angled inwards toward the septum of the nose and are round or elliptical in shape, or in a shape that corresponds to the inner dimensions of said patient's nares.

5. A breathing assistance apparatus according to any one of claims 2 to 4 wherein each of said nasal prongs (40, 41) directs gases flow toward the back of the patient's head, so that the gases flow directly through said patient's nasal passages.

6. A breathing assistance apparatus according to any one of claims 1 to 5 wherein one of said humidification means (1), said humidified gases transport means (6) and said nasal cannula (30) includes a pressure relief valve (81) to ensure gases pressure can be released upon improper fitting of said prongs (40, 41) or sealing of said prongs (40, 41) within said patient's nares:

7. A breathing assistance apparatus according to any one of claims 1 to 6 wherein a significant portion of said gases comprise oxygen gas.

8. A breathing assistance apparatus according to claim 7 wherein said portion is greater then 21%.

9. A breathing assistance apparatus according to claim 1 wherein said heating means (10) comprises a heater wire disposed within, throughout or around said transport means.

10. A breathing assistance apparatus according to any one of claims 1 or 9 wherein said heating means (10) is adapted to maintain said gases at a temperature above the saturation point.

11. A breathing assistance apparatus as claimed in any one of claims 1 to 10 wherein the wide bore nasal prong (40, 41) allows delivery of said humidified gases at flow rates exceeding a patient's peak inspiratory flow.

12. A breathing assistance apparatus as claimed in any one of claims 1 to 10 wherein the wide bore nasal prong (40, 41) allows delivery of said humidified gases at flow rates larger than 30litres per minute.

13. A breathing assistance apparatus as claimed in any one of claims 1 to 12 wherein the wide bore nasal prong (40, 41) allows high flow delivery of gases without turbulent gas flow.

14. A breathing assistance apparatus as claimed in any one of claims 1 to 13 wherein the wide bore nasal prong (40, 41) allows high flow delivery of gases without the supply pressure exceeding 50cmH₂0.

15. A breathing assistance apparatus as claimed in any one of claims 1 to 14 wherein the diameter of the nasal prong (40, 41) at the nare is approximately 6mm.

## Patentansprüche

1. Atmungsunterstützungsgerät, das Folgendes umfasst:
eine unter Druck stehende Gasquelle,
Befeuchtungseinrichtung (1), die im Gebrauch mit der Gasquelle in Flüssigkeitsverbindung steht und im Gebrauch die Gase befeuchtet,
Transporteinrichtung (6) für befeuchtete Gase, die im Gebrauch mit der Befeuchtungseinrichtung (1) in Flüssigkeitsverbindung steht und im Gebrauch die befeuchteten Gase befördert,
Heizeinrichtung (10), die innerhalb der Transporteinrichtung (6) angeordnet ist und im Gebrauch die die Transporteinrichtung (6) durchlaufenden befeuchteten Gase erwärmt, und
Nasenkanüle (30), die im Gebrauch mit der Transporteinrichtung (6) in Flüssigkeitsverbindung steht und die befeuchteten Gase einem Patienten zuführt, wobei die Nasenkanüle (30) mindestens eine Nasengabel (40, 41) mit weitem Innendurchmesser einschließt, die nicht die Nasenlöcher verschließt und die die Zuführung der befeuchteten Gase mit hoher Fließgeschwindigkeit erlaubt und positiven Atemwegsdruck in den Atemwegen des Patienten beim Einatmen und positiven endexspiratorischen Druck beim Ausatmen erzeugt.

2. Atmungsunterstützungsgerät nach Anspruch 1, wobei die Kanüle (30) mit zwei Nasengabeln (40, 41) versehen ist, die keinen Verschluss in den Nasenlöchern des Patienten herbeiführen.

3. Atmungsunterstützungsgerät nach Anspruch 2, wobei die Nasengabeln (40, 41) so geformt sind, dass sie dem Nasengang des Patienten anatomisch korrekt angepasst sind.

4. Atmungsunterstützungsgerät nach einem beliebigen der Ansprüche 2 oder 3, wobei die Nasengabeln (40, 41) einwärts zum Septum der Nase hin angewinkelt sind und eine runde oder elliptische Form aufweisen, oder eine Form, die den inneren Abmessungen der Nasenlöcher des Patienten entspricht.

5. Atmungsunterstützungsgerät nach einem beliebigen der Ansprüche 2 bis 4, wobei jede der Nasengabeln (40, 41) den Gasfluss zur Rückseite des Patientenkopfes hin leitet, so dass die Gase direkt durch die Nasengänge des Patienten fließen.

6. Atmungsunterstützungsgerät nach einem beliebigen der Ansprüche 1 bis 5, wobei eine der Komponenten Befeuchtungseinrichtung (1), Transporteinrichtung (6) für befeuchtete Gase und Nasenkanüle (30) ein Druckentlastungsventil (81) beinhaltet, um sicherzustellen, dass bei unsachgemäßer Anbringung der Gabeln (40, 41) oder Verschluss durch die Gabeln (40, 41) innerhalb der Nasenlöcher des Patienten die Gasdruckentlastung erfolgen kann.

7. Atmungsunterstützungsgerät nach einem beliebigen der Ansprüche 1 bis 6, wobei ein signifikanter Anteil der Gase aus Sauerstoffgas besteht.

8. Atmungsunterstützungsgerät nach Anspruch 7, wobei der Anteil größer als 21 % ist.

9. Atmungsunterstützungsgerät nach Anspruch 1, wobei die Heizeinrichtung (10) einen in der, durch die oder um die Transporteinrichtung angeordneten Heizdraht umfasst.

10. Atmungsunterstützungsgerät nach einem beliebigen der Ansprüche 1 oder 9, wobei die Heizeinrichtung (10) die Gase auf einer Temperatur über dem Sättigungspunkt hält.

11. Atmungsunterstützungsgerät wie in einem beliebigen der Ansprüche 1 bis 10 beansprucht, wobei die Nasengabel (40, 41) mit weitem Innendurchmesser die Zuführung der befeuchteten Gase bei Fördermengen ermöglicht, die den maximalen Inspirationsfluss des Patienten übertreffen.

12. Atmungsunterstützungsgerät wie in einem beliebigen der Ansprüche 1 bis 10 beansprucht, wobei die Nasengabel (40, 41) mit weitem Innendurchmesser die Zuführung der befeuchteten Gase bei Fördermengen über 30 Liter pro Minute ermöglicht.

13. Atmungsunterstützungsgerät wie in einem beliebigen der Ansprüche 1 bis 12 beansprucht, wobei die Nasengabel (40, 41) mit weitem Innendurchmesser die Zuführung von Gasen mit hoher Fließgeschwindigkeit ohne turbulente Gasströmung ermöglicht.

14. Atmungsunterstützungsgerät wie in einem beliebigen der Ansprüche 1 bis 13 beansprucht, wobei die Nasengabel (40, 41) mit weitem Innendurchmesser die Zuführung von Gasen mit hoher Fließgeschwindigkeit ermöglicht, ohne dass der Zuführdruck 50 cm H₂0 überschreitet.

15. Atmungsunterstützungsgerät wie in einem beliebigen der Ansprüche 1 bis 14 beansprucht, wobei der Durchmesser der Nasengabel (40, 41) am Nasenloch ungefähr 6 mm beträgt.

## Revendications

1. Dispositif d'assistance respiratoire, comprenant :
une source de gaz sous pression ;
un moyen d'humidification (1) apte, à l'usage, à être en communication fluide avec ladite source de gaz et apte, à l'usage, à humidifier lesdits gaz ;
un moyen de transport desdits gaz humidifiés (6) apte, à l'usage, à être en communication fluide avec ledit moyen d'humidification (1) et apte, à l'usage, à transporter lesdits gaz humidifiés ;
un moyen de chauffage (10) disposé à l'intérieur dudit moyen de transport (6) et apte, à l'usage, à chauffer lesdits gaz humidifiés pendant qu'ils traversent ledit moyen de transport (6) ; et
une canule nasale (30), apte, à l'usage, à être en communication fluide avec ledit moyen de transport (6) et à administrer lesdits gaz humidifiés à un patient, ladite canule nasale (30) comprenant au moins une branche nasale (40, 41) à large orifice qui ne bouche pas les narines et qui permet l'administration à fort débit desdits gaz humidifiés et crée une pression d'air positive dans les voies respiratoires dudit patient pendant l'inspiration et une pression expiratoire finale positive pendant l'expiration.

2. Appareil d'assistance respiratoire conforme à la revendication 1, où ladite canule (30) est dotée de deux branches nasales (40, 41) qui ne bouchent pas l'intérieur des narines dudit patient.

3. Appareil d'assistance respiratoire conforme à la revendication 2, où lesdites branches nasales (40, 41) ont une forme adaptée à l'anatomie des voies nasales dudit patient.

4. Appareil d'assistance respiratoire conforme à une quelconque des revendications 2 ou 3, où lesdites branches nasales (40, 41) sont orientées vers l'intérieur en direction de la cloison nasale et sont de forme ronde ou elliptique, ou de forme correspondant aux dimensions intérieures des narines dudit patient.

5. Appareil d'assistance respiratoire conforme à une quelconque des revendications 2 à 4, où chacune desdites branches nasales (40, 41) dirige le flux de gaz vers l'arrière de la tête du patient, de manière à ce que les gaz s'écoulent directement dans les voies nasales dudit patient.

6. Appareil d'assistance respiratoire conforme à une quelconque des revendications 1 à 5, où un desdits moyens d'humidification (1), desdits moyens de transport des gaz humidifiés (6) et de ladite canule nasale (30) comprend une soupape de surpression (81) pour assurer que la pression des gaz puisse s'échapper en cas de mauvaise installation desdites branches (40, 41) ou de blocage desdites branches (40, 41) à l'intérieur des narines dudit patient.

7. Appareil d'assistance respiratoire conforme à une quelconque des revendications 1 à 6, où une partie importante desdits gaz comprend du gaz oxygène.

8. Appareil d'assistance respiratoire conforme à la revendication 7, où ladite partie est supérieure à 21%.

9. Appareil d'assistance respiratoire conforme à la revendication 1, où ledit moyen de chauffage (10) comprend un filament de chauffage disposé à l'intérieur, à travers ou autour dudit moyen de transport.

10. Appareil d'assistance respiratoire conforme à une quelconque des revendications 1 ou 9, où ledit moyen de chauffage (10) est apte à maintenir lesdits gaz à une température supérieure au point de saturation.

11. Appareil d'assistance respiratoire conforme à une quelconque des revendications 1 à 10, où l'embout nasal à large orifice (40, 41) permet l'administration desdits gaz humidifiés à des débits dépassant le débit inspiratoire maximum du patient.

12. Appareil d'assistance respiratoire conforme à une quelconque des revendications 1 à 10, où l'embout nasal à large orifice (40, 41) permet l'administration desdits gaz humidifiés à des débits dépassant 30 litres/minute.

13. Appareil d'assistance respiratoire conforme à une quelconque des revendications 1 à 12, où l'embout nasal à large orifice (40, 41) permet l'administration à fort débit desdits gaz sans turbulence de l'écoulement de gaz.

14. Appareil d'assistance respiratoire conforme à une quelconque des revendications 1 à 13, où l'embout nasal à large orifice (40, 41) permet l'administration à fort débit des gaz sans que la pression d'alimentation dépasse 50 cmH₂0.

15. Appareil d'assistance respiratoire conforme à une quelconque des revendications 1 à 14, où le diamètre de l'embout nasal (40, 41) à la narine est d'environ 6 mm.
